# EUROPEAN PATENT APPLICATION

(11) **EP 4 397 754 A1**
(43) Date of publication of application: **10.07.2024**
(21) Application number: 22864534.7
(22) Date of filing: 30.08.2022
(51) Int. Cl.: C12N 9/20, C12P 7/6458, C12N 15/55

(54) **ENZYMATIC AGENT FOR TRANSESTERIFICATION CONTAINING LIPASE AS ACTIVE INGREDIENT**

(30) Priority: 30.08.2021 JP 2021139674
(71) Applicant: Amano Enzyme Inc., Nagoya-shi Aichi 460-8630 (JP)
(72) Inventor: MATSUDA, Kanki, Kakamigahara-shi, Gifu 509-0109 (JP); YANG, Minghong, Kakamigahara-shi, Gifu 509-0109 (JP); KODAMA, Yusaku, Kakamigahara-shi, Gifu 509-0109 (JP); TABUSE, Shiho, Kakamigahara-shi, Gifu 509-0109 (JP)
(74) Representative: Witthoff Jaekel Steinecke Patentanwälte PartG mbB
(86) International application number: PCT/JP2022/032516
(87) International publication number: WO 2023/032952

(57) **Abstract**

It is an object of the present invention to provide an enzyme agent for transesterification, including, as an active ingredient, a lipase having excellent heat resistance and high specificity for the 1,3-positions. According to the present invention, provided is an enzyme agent for transesterification, including, as an active ingredient, a lipase consisting of an amino acid sequence having a sequence identity of 90% or more to the amino acid sequence as set forth in SEQ ID NO: 2 or SEQ ID NO: 4.

## Description

### Technical Field

The present invention relates to an enzyme agent for transesterification, including a lipase as an active ingredient, and the use thereof.

### Background Art

There are two methods for modifying the physical properties of oils and fats, namely, chemical transesterification and enzymatic transesterification. However, since chemical transesterification has a high environmental impact, expectations for enzymatic transesterification are increasing. Some lipases having transesterification ability can be used as enzymes for the enzymatic transesterification of oils and fats.

It is said that lipases derived from Candida sp., Alcaligenes sp., Pseudomonas sp., Humicola sp. (product name: Lipozyme TL IM, manufactured by Novozymes), and the like can be utilized for the enzymatic transesterification of oils and fats. However, there are a few enzymes that can be used for food applications.

Meanwhile, Patent Document 1 discloses *Talaromyces thermophilus-derived* lipase, which is used as a catalyst for the synthetic reaction of biofuel (the synthesis of butyl oleate) and for an esterification reaction. Non-Patent Document 1 discloses *Talaromyces thermophilus-derived* lipase, which is used as a catalyst for the synthetic reaction of biofuel (the synthesis of fatty acid methyl esters).

### Prior Art Documents

### Patent Document

Patent Document 1: Tunisian Patent Application No. TN/P/2011/193

### Non-Patent Document

Non-Patent Document 1: Gene Volume 494, Issue 1, 15 February 2012, Pages 112-118

### Summary of Invention

### Object to be Solved by the Invention

As described above, modification of the physical properties of oils and fats by enzymatic transesterification has been studied. Since oils and fats having stearic acid and palmitic acid as constituent fatty acids are solids at room temperature, it is necessary to allow the oils and fats to react, while maintaining the temperature at their melting point or higher during the reaction. Since lipases generally have low thermal stability, it has been desired to develop a lipase having more excellent heat resistance. Moreover, it has also been desired to develop a lipase having high specificity for the 1,3-positions.

It is an object of the invention to provide an enzyme agent for transesterification, including, as an active ingredient, a lipase having excellent heat resistance and high specificity for the 1,3-positions. Further, it is another object of the present invention to provide a method for producing modified oils and fats, using the above-described enzyme agent for transesterification.

### Means for Solving the Object

The present inventors have conducted intensive studies directed towards achieving the aforementioned objects, and the present inventors have screened a library of 10,000 or more bacterial strains for heat resistant lipases. As a result, the present inventors have found two types of heat resistant lipases derived from a *Talaromyces thermophilus* NBRC31798T strain (which is also referred to as a *"Thermomyces dupontii* NBRC31798T strain" at times), thereby completing the present invention. According to the present invention, the following inventions are provided.

< 1 > An enzyme agent for transesterification, including, as an active ingredient, a lipase consisting of an amino acid sequence having a sequence identity of 90% or more to the amino acid sequence as set forth in SEQ ID NO: 2 or SEQ ID NO: 4.
< 2 > The enzyme agent according to < 1 >, in which the lipase is derived from Talaromyces (also referred to as Thermomyces, at times) sp. bacteria.
< 3 > The enzyme agent according to < 1 > or < 2 >, in which the lipase is derived from *Talaromyces thermophilus* (also referred to as *Thermomyces dupontii,* at times).
< 4 > A method for producing transesterified oils and fats, including allowing the enzyme agent according to any one of < 1 > to < 3 > to act on oils and fats.
< 5 > The method according to < 4 >, in which the enzyme agent is allowed to act on oils and fats at a temperature of 60°C or higher.
< 6 > A lipase consisting of an amino acid sequence having a sequence identity of 99% or more to the amino acid sequence as set forth in SEQ ID NO: 2 or SEQ ID NO: 4, in which the enzyme activity remains even after the lipase is treated in a 50 mM PIPES buffer (pH 7.0) at 70°C for 30 minutes.
< 7 > A method for producing a lipase, including the following steps (1) and (2):
   (1) culturing microorganisms having an ability to generate the lipase according to < 6 >; and
   (2) recovering the lipase from the culture solution and/or the cell mass after completion of the culture.
< 8 > The production method according to < 7 >, in which the microorganisms are a *Talaromyces thermophilus* (also referred to as *Thermomyces dupontii,* at times) NBRC31798T strain.

### Advantageous Effects of Invention

The lipase included in the enzyme agent for transesterification of the present invention has high heat resistance and high specificity for the 1,3-positions.

### Brief Description of Drawings

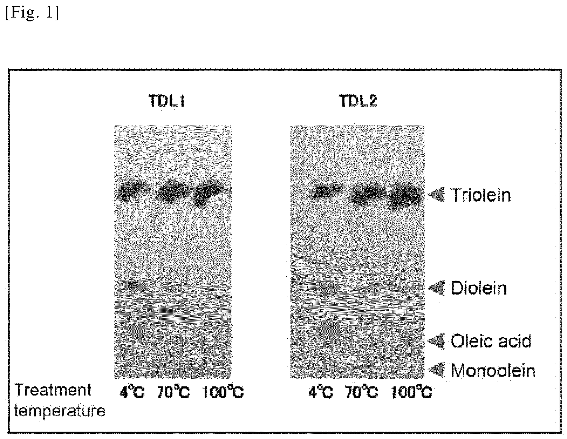

[Fig. 1] Fig. 1 shows the results of heat resistance evaluation.

### Embodiments of Carrying out the Invention

### < 1 > Enzyme agent for transesterification and active ingredient thereof (lipase)

The enzyme agent for transesterification of the present invention includes, as an active ingredient, a lipase consisting of an amino acid sequence having a sequence identity of 90% or more to the amino acid sequence as set forth in SEQ ID NO: 2 or SEQ ID NO: 4.

The lipase used in the present invention is preferably a lipase derived from Talaromyces sp. or Thermomyces sp. bacteria, and is more preferably a lipase derived from *Talaromyces thermophilus* (also referred to as *"Thermomyces dupontii"* at times). The phrase "a lipase derived from *Talaromyces thermophilus* " is used to mean that this lipase is a lipase produced by microorganisms belonging to *Talaromyces thermophilus* (which may be either a wild-type strain or a mutant strain), or a lipase obtained by a genetic engineering method utilizing a lipase gene of *Talaromyces thermophilus* (which may be either a wild-type strain or a mutant strain). Accordingly, mutant lipases produced by host microorganisms, into which a lipase gene obtained from *Talaromyces thermophilus* (or a gene obtained by modification of the lipase gene) or a gene having a nucleotide sequence equivalent thereto has been introduced, also correspond to the lipase derived from *Talaromyces thermophilus.* The *Talaromyces thermophilus,* from which the present enzyme is derived, is referred to as "origin bacteria" for the present enzyme. In addition, the microorganisms used to produce the present enzyme (*Talaromyces thermophilus,* host microorganisms) are referred to as "productive bacteria."

A specific example of such *Talaromyces thermophilus* is a *Talaromyces thermophilus* NBRC31798T strain. The NBRC31798T strain is a bacterial strain preserved at National Institute of Technology and Evaluation (2-5-8, Kazusa Kamatari, Kisarazu-shi, Chiba prefecture, Japan) (this strain is listed as "NBRC31798T" in the NBRC Culture catalog). This strain can be acquired through the predetermined procedures.

The amino acid sequences of the lipases produced by the NBRC31798T strain have been determined (SEQ ID NO: 2 and SEQ ID NO: 4). One aspect of the lipase of the present invention is a protein having the amino acid sequence as set forth in SEQ ID NO: 2 or SEQ ID NO: 4. In general, when the amino acid sequence of a certain protein is partially modified, the modified protein may have functions equivalent to those of the protein before modification in some cases. That is to say, modification of the amino acid sequence does not substantially influence on the functions of the protein, and the functions of the protein may be maintained before and after the modification in some cases. In the present invention, there may be used a protein consisting of an amino acid sequence equivalent to the amino acid sequence as set forth in SEQ ID NO: 2 or SEQ ID NO: 4 and having a transesterification activity (hereinafter also referred to as an "equivalent protein" at times).

The present invention relates to an enzyme agent useful for transesterification of oils and fats (i.e., an enzyme agent for transesterification). The enzyme agent of the present invention (hereinafter also referred to as "the present enzyme agent" at times) includes, as an active ingredient, a transesterification lipase (hereinafter also referred to as "the present enzyme" at times). Such a transesterification lipase serving as an active ingredient, namely, the present enzyme consists of the amino acid sequence as set forth in SEQ ID NO: 2 or SEQ ID NO: 4 or an amino acid sequence equivalent to the aforementioned amino acid sequence.

The term "amino acid sequence equivalent to" means an amino acid sequence that is partially different from a reference amino acid sequence (i.e. the amino acid sequence as set forth in SEQ ID NO: 2 or the amino acid sequence as set forth in SEQ ID NO: 4) but such a difference does not substantially influence on the function of a protein (which is herein transesterification ability). Accordingly, an enzyme having such an equivalent amino acid sequence catalyzes a transesterification reaction. The level of the activity is not particularly limited, as long as the function as a transesterification lipase can be exhibited. The activity of a transesterification lipase having such an equivalent amino acid sequence is preferably the same level as or higher than the activity of the enzyme consisting of a reference amino acid sequence.

Such a "partial difference in the amino acid sequence" is generated, for example, by a deletion or substitution of one or multiple amino acids in the amino acids constituting the amino acid sequence, or by an addition or insertion of one or multiple amino acids with respect to the amino acid sequence, or by any given combination thereof. Such a partial difference in the amino acid sequence is acceptable, as long as transesterification activity is retained (in which some fluctuations in the activity may be acceptable). As long as these conditions are satisfied, the position in which the amino acid sequence is different is not particularly limited. In addition, such differences in the amino acid sequence may be generated in multiple sites (places).

The number of amino acids causing such a partial difference in the amino acid sequence is, for example, a number corresponding to less than about 30%, preferably a number corresponding to less than about 20%, more preferably a number corresponding to less than about 15%, even more preferably a number corresponding to less than about 10%, further preferably a number corresponding to less than about 7%, even further preferably a number corresponding to less than about 5%, still further preferably a number corresponding to less than about 3%, still further preferably a number corresponding to less than about 2%, and most preferably a number corresponding to less than about 1%, of the total amino acids constituting the amino acid sequence. Accordingly, such an equivalent protein has an identity of, for example, about 70% or more, preferably about 80% or more, more preferably about 85% or more, even more preferably about 90% or more, further preferably about 93% or more, even further preferably about 95% or more, still further preferably about 97% or more, still further about 98% or more, and most preferably about 99% or more, to the reference amino acid sequence.

One typical examples of such a "partial difference in the amino acid sequence" is that a mutation (change) is generated by a deletion or substitution of 1 to 40 amino acids (preferably 1 to 30, more preferably 1 to 10, even more preferably 1 to 7, further preferably 1 to 5, and still further preferably 1 to 3 amino acids) in the amino acids constituting the amino acid sequence, or by an addition or insertion of 1 to 40 amino acids (preferably 1 to 30, more preferably 1 to 10, even more preferably 1 to 7, further preferably 1 to 5, and still further preferably 1 to 3 amino acids) with respect to the amino acid sequence, or by a combination thereof.

Preferably, an equivalent amino acid sequence is obtained by generation of a conservative amino acid substitution in amino acid residues that are not essential for transesterification ability. The term "conservative amino acid substitution" is used herein to mean that a certain amino acid residue is substituted with an amino acid residue having a side chain with similar properties. The amino acid residues are classified into several families depending on the side chains thereof, such as basic side chains (e.g. lysine, arginine, and histidine), acidic side chains (e.g. aspartic acid and glutamic acid), uncharged polar side chains (e.g. glycine, asparagine, glutamine, serine, threonine, tyrosine, and cysteine), non-polar side chains (e.g. alanine, valine, leucine, isoleucine proline, phenylalanine, methionine, and tryptophan), β-branched side chains (e.g. threonine, valine, and isoleucine), aromatic side chains (e.g. tyrosine, phenylalanine, tryptophan, and histidine). The conservative amino acid substitution is preferably a substitution between amino acid residues within a single family.

The identity (%) of two amino acid sequences can be determined, for example, by the following procedures. First, two sequences are aligned for optimal comparison (for example, a gap may be introduced into a first sequence to optimize the alignment with a second sequence). If a molecule (amino acid residue) at a specific position in the first sequence is identical to a molecule at the corresponding position in the second sequence, the molecules at that position are said to be identical to each other. The identity of two sequences is a function of the number of identical positions common in the two sequences (i.e., identity (%) = the number of identical positions / the total number of positions x 100), and preferably, the number and size of gaps used for optimization of the alignment are also considered.

Comparison of two sequences and determination of their identity can be realized using mathematical algorithms. A specific example of the mathematical algorithms usable for comparison of sequences is the algorithms that are described in Karlin and Altschul (1990) Proc. Natl. Acad. Sci. USA 87: 2264-68 and are then modified in Karlin and Altschul (1993) Proc. Natl. Acad. Sci. USA 90: 5873-77, but the example of the mathematical algorithms is not limited thereto. The identity of amino acid sequences can be obtained, for example, by blastp (protein-protein BLAST) of National Center for Biotechnology Information (NCBI). As parameters, default parameters may be used. For example, using BLOSUM62 matrix, Gap Costs may be set to be Existence: 11 and Extension: 1.

The transesterification lipase that is an active ingredient of the present enzyme agent, namely, the present enzyme may be a part of a larger protein (for example, a fusion protein). Examples of a sequence to be added to such a fusion protein may include sequences that are useful for purification, such as multiple histidine residues, and additional sequences that ensure stability during recombinant production.

The present enzyme that is an active ingredient of the present enzyme agent catalyzes a transesterification reaction. Transesterification reaction refers to a reaction by which an acid group (carboxylic acid group) in an ester compound is exchanged for another acid (carboxylic acid), and the transesterification reaction is distinguished from an esterification reaction (a reaction to produce an ester from a carboxylic acid and an alcohol). The transesterification reaction in the present invention is a reaction by which fatty acid residues constituting receptor substrates (oils and fats (triglycerides), glycerin fatty acid esters (diglycerides and monoglycerides)) are exchanged with fatty acids of donor substrates (fatty acids and ester compounds (fatty acid esters, etc.)) or oils and fats (which may be the same as those of the receptor substrates)).

In a case where the present enzyme agent is utilized in modification and improvement of the physical properties of oils and fats, oil and fat processed products, etc., it becomes possible to perform transesterification on oils and fats having a high melting point. Thus, it can be expected to improve the quality (to acquire desired physical properties) of such oils and fats, as a result that oils and fats having a fatty acid composition different from that before the treatment (in particular, a composition involving a different fatty acid at position 2) can be obtained.

The content of the active ingredient (the present enzyme) in the present enzyme agent is not particularly limited. For example, the content of the active ingredient can be determined or adjusted, so that the enzyme activity per gram of the present enzyme agent becomes 1 U to 100000 U, and preferably 10 U to 30000 U. The enzyme agent of the present invention is usually provided in a solid form (e.g., granules, powders, or an immobilized enzyme that is immobilized on a material capable of immobilizing the enzyme on a carrier surface or inside of a carrier, such as silica, diatomaceous earth, or a porous polymer), or in a liquid form.

The present enzyme agent may include an excipient, a buffer agent, a suspending agent, a stabilizer, a preservative, an antiseptic, a normal saline, and the like, as well as the active ingredient (the present enzyme). Examples of the excipient that can be used herein may include lactose, sorbitol, D-mannitol, maltodextrin, and white sugar. Examples of the buffer agent that can be used herein may include phosphate, citrate, and acetate. Examples of the stabilizer that can be used herein may include propylene glycol and ascorbic acid. Examples of the preservative that can be used herein may include phenol, benzalkonium chloride, benzyl alcohol, chlorobutanol, and methylparaben. Examples of the antiseptic that can be used herein may include benzalkonium chloride, paraoxybenzoic acid, and chlorobutanol.

The present invention further relates to a lipase consisting of an amino acid sequence having a sequence identity of 99% or more to the amino acid sequence as set forth in SEQ ID NO: 2 or SEQ ID NO: 4, in which the enzyme activity remains even after the lipase is treated in a 50 mM PIPES buffer (pH 7.0) at 70°C for 30 minutes. The present invention further relates to a lipase consisting of the amino acid sequence as set forth in SEQ ID NO: 2 or SEQ ID NO: 4.

### < 2 > Method for producing the present enzyme

The present enzyme can be obtained by culturing microorganisms that generate a transesterification lipase (i.e., a transesterification lipase-generating strain).

The method for producing the lipase of the present invention may include the following steps (1) and (2):
(1) culturing microorganisms having an ability to produce a lipase consisting of an amino acid sequence having a sequence identity of 99% or more to the amino acid sequence as set forth in SEQ ID NO: 2 or SEQ ID NO: 4, in which the enzyme activity remains even after the lipase is treated in a 50 mM PIPES buffer (pH 7.0) at 70°C for 30 minutes; and
(2) recovering the lipase from the culture solution and/or the cell mass after completion of the culture.

The transesterification lipase-generating strain may be either a wild-type strain or a mutant strain (for example, a mutant strain obtained by ultraviolet irradiation). A specific example of the strain of generating the present enzyme is a *Talaromyces thermophilus* NBRC31798T strain. A mutant strain can be obtained by irradiation with ultraviolet rays, X-rays, γ-rays, etc. or by treatment with nitrous acid, hydrokylamine, or N-methyl-N'-nitro-N-nitrosoguanidine. Such a mutant strain is not limited, as long as it generates the present enzyme. Examples of the mutant strain may include those with improved productivity of the present enzyme, those with reduced productivity of foreign matters, those with easier culture, and those with easier recovery from the culture solution.

According to the method for producing the present enzyme, a step of culturing microorganisms of generating the present enzyme (step (1)), and a step of recovering the lipase from the culture solution and/or the cell mass after completion of the culture (step (2)), can be carried out.

The culture conditions or the culture methods are not particularly limited, as long as the present enzyme can be produced thereby. That is to say, the methods or the culture conditions that are adapted to the culture of the used microorganisms can be determined, as appropriate, under conditions in which the present enzyme is produced. Regarding the culture method, either liquid culture or solid culture may be applied, and liquid culture is preferably utilized. Taking the liquid culture as an example, the culture conditions will be described below.

The medium is not particularly limited, as long as it is a medium in which the used microorganisms can grow. Examples of the medium that can be used herein may include those to which the following substances are added: carbon sources, such as glucose, sucrose, gentiobiose, soluble starch, glycerin, dextrin, molasses, and organic acids; nitrogen sources, such as ammonium sulfate, ammonium carbonate, ammonium phosphate, ammonium acetate, or peptone, yeast extract, corn steep liquor, casein hydrolysate, bran, and meat extract; and further, inorganic salts, such as potassium salts, magnesium salts, sodium salts, phosphates, manganese salts, iron salts and zinc salts. In order to promote the growth of the used microorganisms, vitamins, amino acids, and the like may be added to the medium. The pH of the medium is adjusted to, for example, about 3 to 8, preferably about 4 to 7. The culture temperature is usually about 20°C to 40°C, preferably about 25°C to 35°C, and the microorganisms are cultured for 1 to 20 days, and preferably 3 to 10 days, under aerobic conditions. As a culture method applied herein, for example, a shaking culture method, or an aerobic deep culture method using ajar fermenter, can be utilized.

After completion of the culture performed under the above-described conditions, the enzyme of interest is recovered from the culture solution or the culture mass (step (2)). When the enzyme is recovered from the culture solution, for example, the culture supernatant is filtrated, centrifuged, etc. to remove insoluble matters, and thereafter, the resultant is separated and purified by appropriately combining concentration using an ultrafiltration membrane, salting-out such as ammonium sulfate precipitation, dialysis, and various types of chromatography using ion exchange resin, etc., thereby obtaining the present enzyme. On the other hand, when the enzyme is recovered from the cell mass, for example, the cell mass is crushed by a pressure treatment, an ultrasonic treatment, etc., and is then separated and purified in the same manner as that described above, so as to obtain the present enzyme. Besides, the cell mass may be previously recovered from the culture solution by filtration, a centrifugation treatment, etc., and thereafter, the above-described series of steps (crushing, separation, and purification of the cell mass) may be carried out.

Also, the present enzyme can be easily prepared by a genetic engineering technique. For example, the present enzyme can be prepared by transforming suitable host cells (for example, *Escherichia coli*) with DNA encoding the present enzyme (as examples, a DNA sequence encoding the present enzyme consisting of the amino acid sequence of SEQ ID NO: 2 is shown in SEQ ID NO: 1, and a DNA sequence encoding the present enzyme consisting of the amino acid sequence of SEQ ID NO: 4 is shown in SEQ ID NO: 3, respectively), and then recovering a protein expressed in the transformant. The recovered protein is appropriately purified, depending on the purpose. As such, in a case where the present enzyme can be obtained as a recombinant protein, various modifications can be carried out thereon. For example, a DNA encoding the present enzyme and another appropriate DNA are inserted into an identical vector, and a recombinant protein is produced using the vector, so that the present enzyme consisting of a recombinant protein, in which any given peptides or proteins are ligated to each other, can be obtained. In addition, modification may be carried out, so that addition of a sugar chain and/or a lipid or the processing of the N-terminus or the C-terminus may occur. By performing the aforementioned modification, simplification of the extraction and purification of a recombinant protein, or addition of biological functions, etc. can be carried out.

Generally, the expression of a gene and the recovery of a gene expression product (the present enzyme) are carried out, utilizing an appropriate host-vector system, as described above. However, a cell-free synthesis system may also be utilized. Herein, the term "cell-free synthesis system (a cell-free transcription system or a cell-free transcription/translation system)" means that, not using living cells, but using a ribosome, a transcription/translation factor or the like derived from living cells (or obtained by a genetic engineering technique), from a nucleic acid (DNA or mRNA) used as a template, mRNA or a protein encoded by the nucleic acid is synthesized *in vitro.* In general, in the cell-free synthesis system, a cell extract obtained by purifying, as necessary, a cell-disintegrated solution is used. The cell extract generally includes a ribosome, various types of factors such as an initiation factor, and various types of enzymes such as tRNA, which are necessary for protein synthesis. When a protein is synthesized, various types of amino acids, energy sources such as ATP or GTP, and other substances necessary for protein synthesis, such as creatine phosphate, are added to the aforementioned cell extract. As a matter of course, upon the synthesis of a protein, a ribosome, various types of factors, and/or various types of enzymes, and the like, which are prepared separately, may also be added to the aforementioned cell extract, as necessary.

The development of a transcription/translation system, in which various molecules (factors) necessary for protein synthesis have been reconstructed, has also been reported (Shimizu, Y. et al.: Nature Biotech., 19, 751-755, 2001). In this synthesis system, genes of 31 types of factors composed of: 3 types of initiation factors, 3 types of elongation factors, 4 types of factors associated with termination, 20 types of aminoacyl tRNA synthesis enzymes that allow each amino acid to bind to tRNA, and a methionyl tRNA formyl transfer enzyme, which constitute a protein synthesis system of bacteria, are amplified from an *Escherichia coli* genome, and then, using these amplified products, a protein synthesis system is reconstructed *in vitro.* In the present invention, such a reconstructed synthesis system may be utilized.

The term "cell-free transcription/translation system" is exchangeably used with the term "cell-free protein synthesis system," *"in vitro* translation system" or *"in vitro* transcription/translation system." In the *in vitro* translation system, RNA is used as a template to synthesize a protein. As such template RNA, total RNA, mRNA, an *in vitro* transcriptional product, or the like is used. On the other hand, in the *in vitro* transcription/translation system, DNA is used as a template. The template DNA should include a ribosome-binding region, and preferably includes an appropriate terminator sequence. In addition, in the *in vitro* transcription/translation system, in order to promote continuous progression of the transcription reaction and the translation reaction, conditions, in which factors necessary for each reaction are added, are established.

The purified enzyme obtained as described above is pulverized, for example, by freeze drying, vacuum drying, or spray drying, so that the enzyme can be provided in the form of powders. At that time, the purified enzyme may be previously dissolved in an acetate buffer, a phosphate buffer, a triethanolamine buffer, a Tris-HCl buffer, or a GOOD buffer. Preferably, an acetate buffer, a phosphate buffer, or a triethanolamine buffer can be used. Besides, examples of the GOOD buffer used herein may include PIPES, MES, and MOPS.

The purification degree of the enzyme is not particularly limited, and for example, the enzyme can be purified so that a specific activity of hydrolytic activity can be 1 to 100000 (U/µg), and preferably 10 to 20000 (U/µg). In addition, the final form of the enzyme may be either a liquid or a solid (including powders).

### < 3 > Intended use of the present enzyme agent/the present enzyme (transesterification method of oils and fats)

According to the present invention, provided is a method for producing transesterified oils and fats, including allowing the present enzyme agent to act on oils and fats. Specifically, according to the present invention, provided is a transesterification method of oils and fats, in which the present enzyme agent is used. In the transesterification method of the present invention, a step of allowing the present enzyme agent or the present enzyme to act on oils and fats is carried out, and by this step, transesterification of oils and fats is carried out, namely, fatty acids that constitute triacylglycerol (abbreviated as "TG" or "TAG") in oils and fats are reorganized (rearranged).

Examples of the oils and fats that can be treated by the transesterification method of the present invention may include: vegetable oils and fats, such as soybean oil, rape seed oil, rice oil, corn oil, sunflower oil, cottonseed oil, peanut oil, safflower oil, palm oil, palm soft oil, palm fractionated oil, palm kernel oil, coconut oil, and cacao butter; animal oils and fats, such as fish oil, lard, beef tallow, and milk fat; and fractionated oils and hardened oils thereof, and synthetic oils and fats, such as trilaurin, triolein and tripalmitin. The transesterification method of the present invention can be utilized in transesterification between oils and fats and fatty acids or fatty acid esters, as well as transesterification between oils and fats. Examples of the fatty acids used herein may include stearic acid, palmitic acid, lauric acid, arachidic acid, behenic acid, oleic acid, and linoleic acid. Examples of the fatty acid esters used herein may include ethyl stearate, ethyl palmitate, ethyl oleate, and ethyl linoleate.

In the transesterification method of the present invention, the present enzyme agent or the present enzyme is added to oils and fats, and they are then allowed to react under conditions of, for example, 10°C to 110°C, 20°C to 110°C, 30°C to 105°C, 40°C to 105°C, preferably 50°C to 100°C, more preferably 60°C to 100°C, further preferably 70°C to 100°C, and still further preferably 80°C to 100°C, for a certain period of time (for example, 10 minutes to 72 hours, 1 hour to 48 hours, or 2 hours to 24 hours). In order to promote the reaction, the mixture may be stirred during the reaction.

The present enzyme agent or the present enzyme may be subjected to an immobilization treatment, and a reaction with an immobilized enzyme may be performed. For such a reaction with an immobilized enzyme, a batch type stirred tank reactor, a flow-through type stirred tank reactor, a packed-bed reactor, a fluidized-bed reactor, etc. can be utilized.

The transesterification method of the present invention is useful for the modification and improvement of the physical properties of oils and fats or oil and fat processed products (e.g., shortening, margarine, and cacao butter substitute fats). The transesterification method of the present invention can be applied for the purpose of, for example, the improvement of ductility, the improvement of emulsion stability, optimization of a solid fat content (SFC), the improvement of solidification, selective concentration of specific fatty acids, production of low trans-acid oils and fats or low trans-acid oil and fat processed products, etc. The oils and fats obtained by applying the transesterification method of the present invention or oil and fat processed products including them are found to improve their physical properties, compared with those before the treatment, and thus, the obtained oils and fats or the oil and fat processed products have a high value in industrial application.

According to the transesterification method of the present invention, transesterified oils and fats can be produced. That is to say, the present invention also provides a method for producing transesterified oils and fats. Typically, in the method for producing the transesterified oils and fats of the present invention, acceptor substrates (oils and fats (triglycerides), glycerin fatty acid esters (diglycerides and monoglycerides)) and the donor substrates (fatty acids, ester compounds (fatty acid esters, etc.) or oils and fats (which may be the same as those for the acceptor substrate)) are prepared, and the present enzyme agent or the present enzyme is then allowed to act thereon (i.e., an enzyme reaction is carried out in the presence of the acceptor substrates and the donor substrates). As oils and fats, fatty acids, and fatty acid esters, which are used as acceptor substrates and donor substrates, those as described above can be used. As conditions for the enzyme reaction, the above-described conditions can be adopted (i.e., a reaction is carried out under conditions of. for example, 30°C to 100°C, preferably 40°C to 100°C, more preferably 60°C to 100°C, and further preferably 70°C to 90°C, for a certain period of time (for example, 1 hour to 48 hours)). According to one example of the present invention, provided is a method for producing SOS fats (S: stearic acid, O: oleic acid) serving as main components of cacao butter, including a step of preparing triolein (OOO) (O: oleic acid) as an acceptor substrate and stearic acid or methyl stearate as a donor substrate, and a step of allowing the present enzyme agent or the present enzyme to act on the prepared acceptor substrate and donor substrate under conditions of 40°C to 100°C, preferably 60°C to 100°C, and more preferably 70°C to 90°C. SOS fats are triglycerides in which stearic acid (S) and oleic acid (O) bind to glycerin in the order of SOS.

### Examples

### Example 1: Sequence determination

### < Preparation of TDL2 gene probe for use in Southern blotting >

Using the genomic DNA of *Talaromyces thermophilus* NBRC31798T as a template, the primers shown in Table 1 were designed. Thereafter, a TDL2 gene probe was prepared by PCR labeling using PCR DIG Synthesis Kit (manufactured by Roche).

**[Table 1]**

| Table 1. Primer Sequences | |
|---|---|
| Primer F1 | 5'-ATGAGGAGCTCCCTTGTGCTGTTCTTCATCTCTGC-3' (SEQ ID NO: 7) |
| Primer R1 | 5'-TTAATCACACTCTGCAATGGCTCCAAAGTACCAT-3' (SEQ ID NO: 8) |

A sample obtained by treating the genomic DNA of *Talaromyces thermophilus* NBRC31798T with BamHI at 37°C overnight and the TDL2 gene probe were used to perform Southern blotting. As a result, two bands were detected. A band with about 3.0 kbp was assumed to be a fragment including the full-length TDL2 gene. On the other hand, regarding a band with about 5.0 kbp, it was assumed that a fragment including full-length TDL1 was detected. The fragment with about 5.0 kbp and the fragment with about 3.0 kbp, which were assumed to include the TDL1 gene and the TDL2 gene, respectively, were used in a ligation reaction, so that each DNA was self-annulated.

In order to amplify the DNA fragment including the TDL1 gene, using the self-annulated DNA (about 5.0 kbp) as a template, PCR was carried out with the primer F2 and the primer R2 shown in Table 2.

In order to amplify the DNA fragment including the TDL2 gene, using the self-annulated DNA (about 3.0 kbp) as a template, PCR was carried out with the primer F3 and the primer R3 shown in Table 2.

The amplified DNA fragments were purified, and were then subjected to sequence analysis.

**[Table 2]**

| Table 2. Primer Sequences | |
|---|---|
| Primer F2 | 5'-AGGGGTGTCTAATACACAGACCAACAGTTCT-3' (SEQ ID NO: 9) |
| Primer R2 | 5'-GCACATTTCCACATGACCGCTCCACGGGGATCA-3' (SEQ ID NO: 10) |
| Primer F3 | 5'-GGAATCTCATCGTCGGTCTGCGAGATATCGA-3' (SEQ ID NO: 11) |
| Primer R3 | 5'-CGATCCAGTTCTCTATTGAGCGAGAGCCGCGGA-3' (SEQ ID NO: 12) |

The identified sequences of the TDL1 gene and the TDL2 gene are shown in Table 3, and the amino acid sequences translated based on the identified sequences of the TDL1 gene and the TDL2 gene are shown in Table 4.

**[Table 3]**

| Table 3. Sequences of TDL1 Gene and TDL2 Gene |
|---|
| DNA sequence of TDL1 gene (sequence including intron) (SEQ ID NO: 5) |
| ATGAGGAGCTCGCTCGTGCTGTTCTTCGTTTCTGCGTGGACGGCCTTGGCCA GTCCTGTCCGACGAGGTATGTAAATCACGGGGTATACTTTTCATGCATTGCAT GTCGAACCTGCTGTACTAAGATTGCGCGCACAGAGGTCTCGCAGGATCTGTT TGACCAGTTCAACCTCTTTGCGCAGTACTCGGCGGCCGCATACTGCGCGAAG AACAACGATGCCCCGGCAGGTGGGAACGTAACGTGCAGGGGAAGTATTTGC CCCGAGGTAGAGAAGGCGGATGCAACGTTTCTCTACTCGTTTGAGGAGTAGG TGTCAACAAGAGTACAGGCACCCGTAGTAGAAATAGCAGACTAACTGGGAA ATGTAGTTCTGGAGTTGGCGATGTCACCGGGTTCCTTGCTCTCGACAACACG AACAGACTGATCGTCCTCTCTTTCCGCGGCTCTCGTTCCCTGGAAAACTGGAT CGGGAATATCAACTTGGACTTGAAAGGAATTGACGACATCTGCTCTGGCTGC AAGGGACATGACGGCTTCACTTCCTCCTGGAGGTCCGTTGCCAATACCTTGA CTCAGCAAGTGCAGAATGCTGTGAGGGAGCATCCCGACTACCGCGTCGTCTT CACTGGGCACAGCTTGGGTGGTGCATTGGCAACTGTGGCCGGGGCATCTCTG |
| CGTGGAAATGGGTACGATATAGATGTGGTATGTAGGAAAAATGATCCCCGTGG AGCGGTCATGTGGAAATGTGCAGGGGTGTCTAATACACAGACCAACAGTTCT CATATGGCGCTCCCCGCGTCGGAAACAGGGCTTTTGCGGAATTCCTGACCGC ACAGACCGGCGGCACCTTGTACCGCATCACCCACACCAATGATATTGTCCCCA GACTCCCGCCACGCGAATTGGGTTACAGCCATTCTAGCCCAGAGTATTGGATC ACGTCTGGAACCCTCGTCCCAGTGACCAAGAACGATATCGTCAAGGTGGAGG GCATCGATTCCACCGATGGAAACAACCAGCCAAATACCCCGGACATTGCTGC GCACCTATGGTACTTCGGGTCAATGGCGACGTGTTTGTAA |
| DNA sequence of TDL2 gene (sequence including intron) (SEQ ID NO: 6) |
| ATGAGAGGTTTTCTCGTGCTGTTCTTCATCTCTGCGTGGACGGCCTTGGCCAG GCCTGTTCGACGAGGTATGGAATCAAGGGACACTTTCATGCATTGCATGTTGA ACCTGCTGTTCTAAGATTGTTCGCGCAGAAGTCTCGCAGGATCTGCTCGATCT GTTCAACCTCTTTGCACAATACTCGGCAGCCGCCTACTGCGCGGAAAACAAC GACGCTCCAGTGGGCTCGAACGTAACGTGCTCGGAGAATGTCTGTCCCAAGG TAGAGGAGGCGGACGCAACATTCCTCTATTCTTTTGAAGAGTGGGTGTCGAC GAATATACAAAGACAGTACCTAGTCTAACTGGGAAATATGTATATATAGTTCTG GACTGGGCGATGTCACCGGCCTTCTCGCGCTCGACAACACGAACAAACTGAT CGTCCTTGCTTTCCGCGGCTCTCGCTCAATAGAGAACTGGATCGGGAATCTCA TCGTCGGTCTGCGAGATATCGACGATATCTGCTCCGGCTGCGAGGGGCATACC GGCTTCGTGGCTTCCTGGAAGTCCGTAGCCAATACCCTGAGAGGACAGGTCG AGAATGCCGTGAAGGAGCATCCCGACTACCGCGTGGTCTTCACAGGACATAG TTTGGGTGGTGCACTGGCAACCATTGCTGGAGCCGCTCTGCGTGGGAATGGG TATAATATCGACGTGGTATGTGGAAATAGTTCCCCAGACAACGATCATGTGGA AACGTGCAGGGGTGTCTAATACACGGACCAACAGTTCTCATATGGCGCCCCC CGCGTCGGCAACAGGGCTTTTGCAGAATTTCTGACCGCACAGACCGGTGGCA CCCTGTACCGCATCACCCACACCAATGATATTGTCCCCAGACTCCCACCGCGA GACTGGGGTTACAGTCACTCCAGCCCAGAGTACTGGATCACGTCCGGCAATG ACGCCCCAGTGACCGCAAACGATATCGTCAAGGTGGAGGGCATTGATTCCAC CGACGGTAACAACCAGCCGAATATCCCGGACATTCCTTCACATCTATGGTACT |
| TTGGAGCCATTGCAGAGTGTGATTAA |

**[Table 4]**

| Table 4. Amino Acid Sequences of TDL1 and TDL2 |
|---|
| Amino acid sequence of TDL1 (SEQ ID NO: 2) |
| MRSSLVLFFVSAWTALASPVRREVSQDLFDQFNLFAQYSAAAYCAKNNDAPAG GNVTCRGSICPEVEKADATFLYSFEDSGVGDVTGFLALDNTNRLIVLSFRGSRSL ENWIGNINLDLKGIDDICSGCKGHDGFTSSWRSVANTLTQQVQNAVREHPDYRV VFTGHSLGGALATVAGASLRGNGYDIDVFSYGAPRVGNRAFAEFLTAQTGGTLY RITHTNDIVPRLPPRELGYSHSSPEYWITSGTLVPVTKNDIVKVEGIDSTDGNNQP NTPDIAAHLWYFGSMATCL |
| Amino acid sequence of TDL2 (SEQ ID NO: 4) |
| MRGFLVLFFISAWTALARPVRREVSQDLLDLFNLFAQYSAAAYCAENNDAPVGS NVTCSENVCPKVEEADATFLYSFEDSGLGDVTGLLALDNTNKLIVLAFRGSRSIE NWIGNLIVGLRDIDDICSGCEGHTGFVASWKSVANTLRGQVENAVKEHPDYRVV FTGHSLGGALATIAGAALRGNGYNIDVFSYGAPRVGNRAFAEFLTAQTGGTLYRI THTNDIVPRLPPRDWGYSHSSPEYWITSGNDAPVTANDIVKVEGIDSTDGNNQP NIPDIPSHLWYFGAIAECD |

On the basis of the identified heat resistant lipase genes, cDNA sequences were deducted using FGENESH (FGENESH - HMM-based gene structure prediction (softberry.com)).

cDNA sequence of TDL1 (SEQ ID NO: 1) 
ATGAGGAGCTCGCTCGTGCTGTTCTTCGTTTCTGCGTGGACGGCCTTGGCCAGT
CCTGTCCGACGAGAGGTCTCGCAGGATCTGTTTGACCAGTTCAACCTCTTTGCG
CAGTACTCGGCGGCCGCATACTGCGCGAAGAACAACGATGCCCCGGCAGGTGG
GAACGTAACGTGCAGGGGAAGTATTTGCCCCGAGGTAGAGAAGGCGGATGCAA
CGTTTCTCTACTCGTTTGAGGATTCTGGAGTTGGCGATGTCACCGGGTTCCTTG
 
CTCTCGACAACACGAACAGACTGATCGTCCTCTCTTTCCGCGGCTCTCGTTCCC
TGGAAAACTGGATCGGGAATATCAACTTGGACTTGAAAGGAATTGACGACATC
TGCTCTGGCTGCAAGGGACATGACGGCTTCACTTCCTCCTGGAGGTCCGTTGCC
AATACCTTGACTCAGCAAGTGCAGAATGCTGTGAGGGAGCATCCCGACTACCG
CGTCGTCTTCACTGGGCACAGCTTGGGTGGTGCATTGGCAACTGTGGCCGGGG
CATCTCTGCGTGGAAATGGGTACGATATAGATGTGTTCTCATATGGCGCTCCCCG
CGTCGGAAACAGGGCTTTTGCGGAATTCCTGACCGCACAGACCGGCGGCACCT
TGTACCGCATCACCCACACCAATGATATTGTCCCCAGACTCCCGCCACGCGAAT
TGGGTTACAGCCATTCTAGCCCAGAGTATTGGATCACGTCTGGAACCCTCGTCC
CAGTGACCAAGAACGATATCGTCAAGGTGGAGGGCATCGATTCCACCGATGGA
AACAACCAGCCAAATACCCCGGACATTGCTGCGCACCTATGGTACTTCGGGTCA
ATGGCGACGTGTTTGTAA
cDNA sequence of TDL2 (SEQ ID NO: 3)

ATGAGAGGTTTTCTCGTGCTGTTCTTCATCTCTGCGTGGACGGCCTTGGCCAGG
CCTGTTCGACGAGAAGTCTCGCAGGATCTGCTCGATCTGTTCAACCTCTTTGCA
CAATACTCGGCAGCCGCCTACTGCGCGGAAAACAACGACGCTCCAGTGGGCTC
GAACGTAACGTGCTCGGAGAATGTCTGTCCCAAGGTAGAGGAGGCGGACGCA
ACATTCCTCTATTCTTTTGAAGATTCTGGACTGGGCGATGTCACCGGCCTTCTCG
CGCTCGACAACACGAACAAACTGATCGTCCTTGCTTTCCGCGGCTCTCGCTCAA
TAGAGAACTGGATCGGGAATCTCATCGTCGGTCTGCGAGATATCGACGATATCT
GCTCCGGCTGCGAGGGGCATACCGGCTTCGTGGCTTCCTGGAAGTCCGTAGCC
AATACCCTGAGAGGACAGGTCGAGAATGCCGTGAAGGAGCATCCCGACTACCG
CGTGGTCTTCACAGGACATAGTTTGGGTGGTGCACTGGCAACCATTGCTGGAG
CCGCTCTGCGTGGGAATGGGTATAATATCGACGTGTTCTCATATGGCGCCCCCCG
CGTCGGCAACAGGGCTTTTGCAGAATTTCTGACCGCACAGACCGGTGGCACCC
TGTACCGCATCACCCACACCAATGATATTGTCCCCAGACTCCCACCGCGAGACT
GGGGTTACAGTCACTCCAGCCCAGAGTACTGGATCACGTCCGGCAATGACGCC
CCAGTGACCGCAAACGATATCGTCAAGGTGGAGGGCATTGATTCCACCGACGG
TAACAACCAGCCGAATATCCCGGACATTCCTTCACATCTATGGTACTTTGGAGCC

ATTGCAGAGTGTGATTAA

### Example 2: Evaluation of heat resistance

### < Preparation of purified enzymes >

*Talaromyces thermophilus* NBRC31798T was subjected to a solid culture (cultured at 40°C for 4 days) in an autoclaved wheat bran medium, and the obtained culture solution was then filtrated. Thereafter, the resultant was subjected to anion exchange chromatography (HiPrep DEAE FF, manufactured by GE Healthcare) under linear gradient conditions of a 20 mM phosphate buffer (pH 8.0) and a 20 mM phosphate buffer (pH 8.0) containing 1.0 M sodium chloride. After that, the resultant was subjected to hydrophobic chromatography (Hitrap Butyl FF, manufactured by GE Healthcare) under linear gradient conditions of a 20 mM phosphate buffer (pH 7.0) containing 1.0 M ammonium sulfate and a 20 mM phosphate buffer (pH 7.0), so as to obtain purified enzymes.

### < Heat treatment >

The purified enzymes of TDL1 and TDL2 were 2-fold diluted with a 50mM PIPES buffer (pH 7.0) containing cOmplete ULTRA Tablets (Protein Inhibitor, manufactured by Roche), and were then subjected to a heat treatment (70°C or 100°C for 30 minutes) in a water bath, so as to prepare heat-treated samples.

### Example 3: Method of confirming triolein hydrolyzing activity of lipase

### < Method >

Triolein was separated from sunflower oil, using a silica gel column, and was then mixed with a polyvinyl alcohol solution for emulsification. Each sample was added to the emulsified triolein solution, and a hydrolysis reaction (40°C, 1 hour) was then carried out. Thereafter, ethyl acetate was added to the reaction solution, and triolein and reaction products (diolein, monoolein, and oleic acid) were then extracted. The extracts were separated and detected by thin layer chromatography under conditions of a developing solvent of chloroform : acetone = 96 : 4.

### < Results >

As a result of the heat resistance evaluation (Fig. 1), since reaction products (diolein and oleic acid) could be confirmed even in the TDL1 sample treated at 70°C, it was revealed that the activity remained even after the treatment at 70°C for 30 minutes.

On the other hand, in the case of TDL2, since reaction products (diolein and oleic acid) could be confirmed even in the TDL2 samples treated at 70°C and 100°C, the activity remained even after the treatments at 70°C and 100°C for 30 minutes, and thus, TDL2 could be confirmed to have high heat resistance.

### Example 4: Confirmation of transesterification activity of lipase

### < Obtaining of recombinant enzyme-expressing strain >

According to PCR using the genomic DNA of *Talaromyces thermophilus* NBRC31798T (*Thermomyces dupontii* NCBI: txid28565) as a template, the TDL1 gene (SEQ ID NO: 5) and the TDL2 gene (SEQ ID NO: 6) were amplified. The amplified DNA was linked to a filamentous fungus expression vector (pUC19, into which a modified promoter of α-amylase derived from *Aspergillus oryzae,* a terminator of FAD-GDH derived from *Aspergillus oryzae,* and a pyrG gene had been inserted), and the thus obtained vector was then introduced into *A*. *oryzae* according to a protoplast-PEG method.

### < Method of obtaining recombinant enzyme >

The obtained transformant was seeded into a liquid medium (pH 6.0) containing starch, yeast extract and inorganic salts, and was then subjected to an aerated agitation culture at 30°C for 90 hours. Thereafter, the culture supernatant was filtrated through diatomaceous earth to remove the productive bacterial cell mass. The resultant was concentrated with an ultrafiltration membrane, and was then freeze-dried to obtain dry powders of the enzyme.

### < Method of preparing immobilized enzyme >

Diatomaceous earth was used as an immobilization carrier. The amount of a protein in the dry powders of the enzyme was measured using Bradford Method Kit (BIO-RAD), and the dry powders of the enzyme were then dissolved in 1 mL of purified water to prepare an enzyme solution with a protein amount of 0.1 g, based on the measured value. The enzyme solution was immobilized on an immobilization carrier (Celite No.535 (manufactured by Celite)) according to an ordinary method to prepare immobilized enzymes (TDL1-IM and TDL2-IM).

### < Evaluation method >

To a 50-mL tube, 3.4 g of triolein (Olein Rich, manufactured by Showa Sangyo Co., Ltd.) and 5 g of methyl palmitate (manufactured by Wako) were added, and the tube was then incubated at reaction temperatures (40°C and 80°C) for 30 minutes. Lipozyme TL-IM (Novozymes), TDL1-IM, and TDL2-IM were used as immobilized enzymes. After 0.05 g of each immobilized enzyme was added, a reaction was carried out by shaking. After 20 hours, the supernatant was recovered and was used as a reaction sample.

The components contained in the reaction sample were analyzed by gas chromatography (GC). To a 1.5-mL tube, 990 µL of hexane and 10 µL of the reaction sample were added, and the obtained mixture was fully stirred using a vortex mixer. The reaction mixture was filtrated through a 0.45-µm membrane filter to obtain a sample for GC analysis. The GC analysis was carried out under the following conditions. The ratio of methyl oleate (OAME) in all of the components was calculated from the area. A large amount of OAME produced suggests that the transesterification reaction progressed and the transesterification activity was high.
Apparatus used: Agilent 8890 GC
Column: DB-1HT (5 m, 0.25 mm, 0.10 µm)
Injection port temperature: 370°C
Amount injected: 1 µL
Split ratio: 50
Detector temperature: 370°C
Temperature-increasing conditions:

**[Table 5]**

| Rate (°C/min) | Temperature (°C) | Hold (min) |
|---|---|---|
| - | 120 | 4 |
| 20 | 150 | - |
| 30 | 315 | 3 |
| 1.5 | 325 | - |
| 30 | 370 | 2 |

Furthermore, the ratio between POP (in which palmitic acid is ester-bonded to positions 1 and 3 of glycerin and oleic acid is ester-bonded to position 2 of glycerin) and PPO (in which palmitic acid is ester-bonded to positions 1 and 2 of glycerin and oleic acid is ester-bonded to position 3 of glycerin) in the triglycerides contained in the reaction sample was analyzed by HPLC. To a vial, 0.9 mL of hexane and 0.1 mL of a GC analysis sample were added, and the obtained mixture was fully stirred using a vortex mixer. Thereafter, HPLC analysis was carried out under the following conditions. The POP percentage (%) was calculated from the area of POP and PPO according to the formula: POP / (POP + PPO). A high POP percentage suggests high specificity for the 1,3-positions.
Apparatus used: HPLC (Shimadzu)
Column: Silver column KANTO 250-4.6 (5 µm)
Column temperature: 25°C
Eluent: Acetone/Hexane = 3/97
Flow rate: 1.0 mL/min
Detection: ELSD-LT II (350 kPa, 40°C, gain 1)
Amount injected: 20 µL

### < Results >

Generation of OAME could be observed in all of the lipases. TDL1-IM and Lipozyme TL-IM produced an equivalent amount of OAME, while TDL2-IM produced about 60% as much as the other two lipases. The POP percentage (%) obtained using each lipase is shown in Table 6.

**[Table 6]**

| Reaction Temperature | POP / (POP + PPO) (%) | | |
|---|---|---|---|
| | Lipozyme TL-IM | TDL1-IM | TDL2-IM |
| 40°C | 94 | 89 | 99 |
| 80°C | 77 | 92 | 95 |

Both TDL1-IM and TDL2-IM exhibited a high POP percentage at 80°C. It was suggested that TDL1-IM and TDL2-IM had high specificity for the 1,3-positions in the high-temperature reaction, compared with Lipozyme TL-IM.

In order to evaluate the specificity for the 1,3-positions when the amount of OAME produced is comparable, the reaction was carried out with each lipase so that the amount of OAME produced became about 20%, and the POP percentage was then evaluated. The reaction was carried out at a reaction temperature of 80°C by a method equivalent to the above-described evaluation method. The amount of enzyme added and the reaction time were adjusted as appropriate, and then, the supernatant was recovered when the amount of OAME produced reached about 20%.

The POP percentage (%) obtained in the case of using each lipase is shown in Table 2.

**[Table 7]**

| | POP / (POP + PPO) (%) |
|---|---|
| Lipozyme TL-IM | 77 |
| TDL1-IM | 92 |
| TDL2-IM | 86 |

Both TDL1-IM and TDL2-IM showed a higher POP percentage than Lipozyme TL-IM, even when the amount of OAME produced was comparable.

### Industrial Applicability

The enzyme agent of the present invention is suitably used in the fields of food products and medical uses, and thus, the present enzyme agent has a high value in industrial application. In particular, the enzyme agent for transesterification of the present invention, which includes a lipase having high heat resistance and high specificity for the 1,3-positions, is useful for production of cacao butter substitute fats.

The present invention is not limited in any way to the above-described embodiments and examples of the present invention. Various modifications are also included in the present invention, in such a range that those skilled in the art can readily conceive of them, and that the modifications are not deviated from the scope of the claims. The contents of the study papers, laid-open patent publications, patent publications, etc., which are explicitly mentioned in the present description, are cited herein by reference in their entirety.

## Claims

1. An enzyme agent for transesterification, comprising, as an active ingredient, a lipase consisting of an amino acid sequence having a sequence identity of 90% or more to the amino acid sequence as set forth in SEQ ID NO: 2 or SEQ ID NO: 4.

2. The enzyme agent according to claim 1, wherein the lipase is derived from Talaromyces (also referred to as Thermomyces, at times) sp. bacteria.

3. The enzyme agent according to claim 1 or 2, wherein the lipase is derived from *Talaromyces thermophilus* (also referred to as *Thermomyces dupontii,* at times).

4. A method for producing transesterified oils and fats, comprising allowing the enzyme agent according to any one of claims 1 to 3 to act on oils and fats.

5. The method according to claim 4, wherein the enzyme agent is allowed to act on oils and fats at a temperature of 60°C or higher.

6. A lipase consisting of an amino acid sequence having a sequence identity of 99% or more to the amino acid sequence as set forth in SEQ ID NO: 2 or SEQ ID NO: 4, wherein the enzyme activity remains even after the lipase is treated in a 50 mM PIPES buffer (pH 7.0) at 70°C for 30 minutes.

7. A method for producing a lipase, comprising the following steps (1) and (2):
(1) culturing microorganisms having an ability to generate the lipase according to claim 6; and
(2) recovering the lipase from the culture solution and/or the cell mass after completion of the culture.

8. The production method according to claim 7, wherein the microorganisms are a *Talaromyces thermophilus* (also referred to as *Thermomyces dupontii,* at times) NBRC31798T strain.
